(19) Europäisches Patentamt
European Patent Office
Office européen des brevets

(11) **EP 4 446 973 A1**

(12) **EUROPEAN PATENT APPLICATION**
published in accordance with Art. 153(4) EPC

(43) Date of publication:
**16.10.2024  Bulletin 2024/42**

(21) Application number: **22903397.2**

(22) Date of filing: **05.12.2022**

(51) International Patent Classification (IPC):
**G06T 5/00** (2024.01)

(52) Cooperative Patent Classification (CPC):
**A61B 6/5264; A61B 6/503;** A61B 6/032;
A61B 6/037

(86) International application number:
**PCT/CN2022/136636**

(87) International publication number:
**WO 2023/103975 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.12.2021  US 202163286133 P**

(71) Applicant: **Ko, Chi-Lun**
**Taipei 10617 (TW)**

(72) Inventors:
• **YEN, Ruoh-Fang**
  **Taipei Taiwan 10617 (CN)**
• **CHENG, Mei-Fang**
  **Taipei Taiwan 10617 (CN)**
• **KO, Chi-Lun**
  **Taipei Taiwan 10617 (CN)**

(74) Representative: **Gill Jennings & Every LLP**
**The Broadgate Tower**
**20 Primrose Street**
**London EC2A 2ES (GB)**

(54) **MEDICAL IMAGE MOVEMENT DETECTION AND CORRECTION METHOD AND SYSTEM, AND COMPUTER READABLE MEDIUM**

(57)    A method, a system, and a computer-readable medium for motion detection and correction of a medical image are provided, including segmenting a medical image about a target organ into a plurality of frame images in accordance with list mode data, and analyzing a plurality of centers of mass of a volume of interest in the plurality of frame images to compute a motion curve of the target organ during scanning, and executing a reconstruction optimization of the medical image based on the motion curve, and therefore being able to take into account the movement of a human organ or a lesion without installing an additional monitoring device in order to perform motion detection and correction of the medical image.

FIG. 4

**Description**

BACKGROUND

1. Technical Field

The present disclosure relates to a medical imaging technology, and in particular to a method, a system and a computer-readable medium for motion detection and correction of a medical image.

2. Description of Related Art

In the field of precision medicine, nuclear medicine is an indispensable program due to its ability to provide functional assessment. However, the long examination time of nuclear medicine and the movement of a patient's body or organs during the scanning process often lead to blurred and inaccurate images and errors in judgment by physicians.

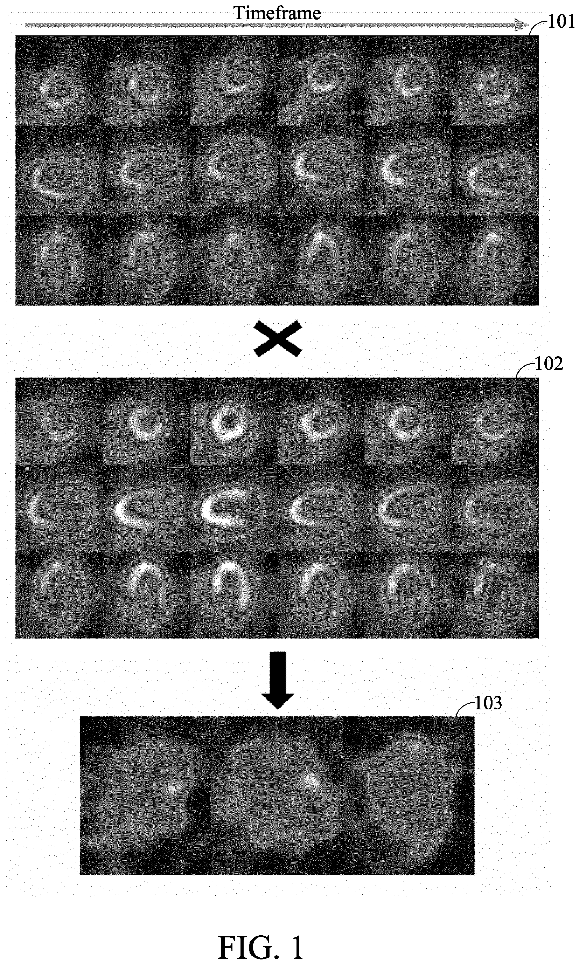

Take myocardial perfusion imaging (MPI) in nuclear medicine as an example, it has the highest utilization rate but is most susceptible to patient mobility. As shown in FIG. 1, due to the contraction and beating behavior of the patient's heart (as shown in FIG. 1, reference numeral 101, which illustrates from left to right the cross-sectional morphology of the heart in the short-axis, vertical-long-axis, and horizontal-long-axis dimensions, respectively, during a cardiac cycle) and the ups and downs of the human body's breathing (as shown in FIG. 1, reference numeral 102, which shows from left to right the cross-sectional morphology of the heart in the short-axis, vertical-long-axis, and horizontal-long-axis dimensions, respectively, during the respiratory cycle), the resulting MPI image of the heart (as shown in FIG. 1, reference numeral 103) is often ambiguous and not easily diagnosed under the influence of both.

In the current technology, the above issues are solved by installing an additional monitoring device to monitor the heartbeat and respiratory displacement of the human body, and then correcting the medical image (e.g., the MPI image) based on the signals measured by the monitoring device. However, such a correction method not only consumes detection resources, but also may result in poor setup of the monitoring device, tracking errors, or failure to integrate with the scanning devices (e.g., commercially available single photon emission computerized tomography [SPECT] devices do not have an optional respiratory monitoring device).

Therefore, how to take into account the movement of human organs or lesions to detect and correct the movement of medical images without installing additional monitoring devices has become one of the pressing issues in this field.

SUMMARY

In order to solve the above problems, the present disclosure provides a system of motion detection and correction of a medical image, the system comprises: a management platform providing a user interface to submit instructions for performing optimization processing on a medical image of a target organ, said medical image comprising list mode data; and an optimization device performing said optimization processing of said medical image in accordance with said instructions, wherein performing optimization processing by said optimization device comprises: segmenting said list mode data corresponding to said medical image into frames having a fixed time dimension to image said frames as a frame image; labeling a volume of interest in each frame image, wherein each volume of interest comprises a target organ; calculating a motion curve of said target organ based on each volume of interest of each frame image; reconstructing said medical image as an optimized medical image based on said motion curve; and displaying said optimized medical image on a user interface.

In at least one embodiment, according to the system of the present disclosure, said optimization device comprises a deep learning module, and performing labeling the volume of interest in each frame image by said optimization device comprises: recognizing a binary segmented volume containing a target organ in each frame image by said deep learning module; blurring each binary segmented volume by said deep learning module to generate a soft mask; applying each soft mask to each said frame image by said deep learning module; fitting the target organ in each frame image with an initial elliptical sphere based on each soft mask by said deep learning module; and expanding each initial elliptical sphere by a predetermined distance outwardly from a radius thereof by said deep learning module to generate an elliptical sphere representing each volume of interest.

In at least one embodiment, according to the system of the present disclosure, performing calculating the motion curve of said target organ based on each volume of interest of each frame image by said optimization device comprises: dividing each volume of interest into a first sub-volume of interest and a second sub-volume of interest; extracting three-dimensional coordinates of a first center of mass (COM) of said first sub-volume of interest of and a second center of mass of said second sub-volume of interest, respectively, to be used as a descriptive value of each frame image; down dimensionalizing each descriptive value of each frame image by principal component analysis, and using the largest feature of each descriptive value after down dimensionalizing as a movement/rotation signal of said target organ; and grouping and filtering each frame image based on each movement/rotation signal to calculate a motion curve.

In at least one embodiment, according to the system of the present disclosure, said target organ is a heart, and said dividing said volume of interest into a first sub-volume of interest and a second sub-volume of interest is along a long axis of said heart in a short-axis direction.

In at least one embodiment, according to the system of the present disclosure, said motion curve is plotted relative to any one of a head-tail axis, a left-right axis, and a ventral-dorsal axis of a human body, and performing reconstructing said medical image into an optimized medical image based on said motion curve by said optimization device comprises: selecting a reference object from each frame image; performing motion compensation of each frame image using said motion curve as a reference, wherein said motion compensation of each frame image comprises: performing an adjustment operation of all pixels contained in each frame image other than said reference object in any of a head-tail axis, a left-right axis, and a ventral-dorsal axis according to three-dimensional coordinates thereof with respect to said reference object; and repeating said adjustment operation of each frame image until correlation coefficient between integration of each frame image after said adjustment operation and said reference object reaching a maximum value; and integrating each frame image after said motion compensation to reconstruct said optimized medical image. In at least one specific embodiment of the present disclosure, said adjustment operation comprises, but is not limited to, rotation, displacement, scaling, deformation, or any combination of more than two of these; in some specific embodiments of the present disclosure, said adjustment operation is translation, rotation, or a combination thereof.

In at least one embodiment, according to the system of the present disclosure, after calculating the motion curve of said target organ based on each center of mass, and each volume of interest of each frame image does not accurately correspond to said motion curve, performing the optimization processing by said optimization device further comprising performing gating of each frame image, wherein said gating is used to assemble a predetermined number of gated set images with similar time and/or positional relationships among said frame images, and said time and/or positional relationships are defined by cyclic phases of human respiration and/or heartbeat.

In at least one embodiment, according to the system of the present disclosure, said motion curve is plotted relative to any one of a head-tail axis, a left-right axis, and a ventral-dorsal axis of a human body, and reconstructing said medical image into said optimized medical image based on said motion curve by said optimization device comprises: selecting a reference object from each said gated set image; performing motion compensation of each said gated set image using said motion curve as a reference, wherein said motion compensation of each said gated set image comprises: performing an adjustment operation of all pixels contained in each said gate set image other than said reference object in any one of a head-tail axis, a left-right axis, and a ventral-dorsal axis according to three-dimensional coordinates thereof with respect to said reference object; and repeating said adjustment operation of each said gated set image until correlation coefficient between integration of each said gate set image after said adjustment operation and said reference object reaching a maximum value; and integrating each gated set image after said motion compensation to reconstruct an optimized medical image.

In at least one embodiment, according to the system of the present disclosure, said fixed time dimension is measured in units of 100 milliseconds to 500 milliseconds.

In at least one embodiment, according to the system of the present disclosure, the present disclosure further comprises: a scanning device photographing a target organ to obtain a medical image, wherein said scanning device is any one of a single photon emission computed tomography device, a positron emission tomography (PET) device, a magnetic resonance imaging (MRI) device, and a computed tomography (CT) device; a picture archiving and communication system (PACS) for storing said medical image and said optimized medical image; and an in-office reporting computer for accessing or displaying said medical image and said optimized medical image.

The present disclosure further provides a method of motion detection and correction of a medical image, the method comprises: obtaining a medical image about a target organ, wherein said medical image comprises list mode data; segmenting said list mode data corresponding to said medical image into frames having a fixed time dimension, and imaging each said frame as a multi-frame image; labeling a volume of interest in each frame image, wherein each volume of interest comprises a target organ; calculating a motion curve of a target organ based on said volume of interest in each frame image; and reconstructing said medical image into an optimized medical image based on the motion curve.

In at least one embodiment, according to the method of the present disclosure, labeling said volume of interest in each frame image comprises: recognizing a binary segmented volume containing said target organ in each frame image by a deep learning module; blurring each binary segmented volume by said deep learning module to generate a soft mask; applying each soft mask to each frame image by said deep learning module; fitting said target organ in each frame image with an initial elliptical sphere based on each soft mask by said deep learning module; and expanding each initial elliptical sphere by a predetermined distance outwardly from a radius thereof by said deep learning module to generate an elliptical sphere representing said volume of interest.

In at least one embodiment, according to the method of the present disclosure, calculating the motion curve of said target organ based on each volume of interest of each frame image comprises: dividing said volume of interest into a first sub-volume of interest and a second sub-volume of interest; extracting three-dimensional coordinates of a first center of mass of each first sub-volume of interest and a second center of mass of each second sub-volume of interest, respectively, to be used as a descriptive value of each frame image; down dimensionalizing said descriptive value of each frame image by principal component analysis method, and using the largest feature of each descriptive value after down dimensionalizing as a movement/rotation signal of said target organ; and grouping and filtering each frame image based on each movement/rotation signal to calculate said motion curve.

In at least one embodiment, according to the method of the present disclosure, said target organ is a heart, and said dividing said volume of interest into a first sub-volume of interest and a second sub-volume of interest is along a long axis of said heart in a short-axis direction.

In at least one embodiment, according to the method of the present disclosure, said motion curve is plotted relative to any one of a head-tail axis, a left-right axis, and a ventral-dorsal axis of a human body, and reconstructing said medical image as an optimized medical image based on said motion curve comprises: selecting a reference object from each frame image; performing motion compensation of each frame image using said motion curve as a reference, wherein said motion compensation of each frame image comprises: performing an adjustment operation of all pixels contained in each frame image other than said reference object in any one of a head-tail axis, a left-right axis, and a ventral-dorsal axis according to three-dimensional coordinates thereof with respect to said reference object; and repeating said adjustment operation of each frame image until correlation coefficient between integration of each frame image after said adjustment operation and said reference object reaching a maximum value; and integrating each frame image after said motion compensation to reconstruct said optimized medical image.

In at least one embodiment, according to the method of the present disclosure, the present disclosure further comprises after calculating the motion curve of a target organ based on each center of mass, and each volume of interest of each frame image does not accurately correspond to said motion curve, gating of each frame image is performed, wherein said gating is used to assemble a predetermined number of gated set images with similar time and/or positional relationships among said frame images, and said time and/or positional relationships are defined by cyclic phases of human respiration and/or heartbeat.

In at least one embodiment of the method of the present disclosure, said motion curve is plotted relative to any one of a head-tail axis, a left-right axis, and a ventral-dorsal axis of a human body, and reconstructing said medical image as an optimized medical image based on said motion curve comprises: selecting a reference object from each said gated set image; and performing motion compensation of each said gated set image using said motion curve as a reference, wherein said motion compensation of each gated set image comprises: performing an adjustment operation of all pixels contained in each said gated set image other than said reference object in any one of a head-tail axis, a left-right axis, and ventral-dorsal axis according to three-dimensional coordinates thereof with respect to said reference object; and repeating said adjustment operation of each said gated set image until correlation coefficient between integration of each said gated set image after said adjustment operation and said reference object reaching a maximum value; and integrating each said gated set image after said motion compensation to reconstruct an optimized medical image.

In at least one embodiment, according to the method of the present disclosure, said medical image is obtained by photographing said target organ by a scanning device, and said scanning device is selected from a group consisting of a single photon emission computed tomography device, a positron emission tomography device, a magnetic resonance imaging device, and a computed tomography device.

In at least one embodiment, according to the method of the present disclosure, said fixed time dimension is measured in units of 100 milliseconds to 500 milliseconds.

The present disclosure further provides a computer-readable storage medium applied in a computer and having instructions stored thereon to perform the method of motion detection and correction of at least one of the above-described medical images.

In summary, the method, system, and computer-readable medium of the present disclosure for motion detection and correction of a medical image can segment a medical image of a target organ into a plurality of frame images in accordance with list mode data, analyze a plurality of centers of mass of a volume of interest in the plurality of frame images to calculate a motion curve of the target organ during a scanning period, and then optimize the reconstruction of the medical image based on the motion curve, and thus can optimize the reconstruction of the medical image. Accordingly, the present disclosure enables motion detection and correction of a medical image by taking into account the movement of human organs or lesions without the need to install an additional monitoring device.

BRIEF DESCRIPTION OF THE DRAWINGS

Specific embodiments of the present disclosure will be detailed with the following drawings. These descriptions are shown in the following drawings.

FIG. 1 is an embodiment of performing myocardial perfusion imaging with current technology.

FIG. 2 is a schematic diagram showing a system architecture for motion detection and correction of a medical image according to the present disclosure.

FIG. 3 is a schematic diagram showing a system of motion detection and correction of a medical image according to an embodiment of the present disclosure.

FIG. 4 is a flowchart of steps showing a method of motion detection and correction of a medical image according to the present disclosure.

FIG. 5 is a partial embodiment showing a method of motion detection and correction of a medical image according to the present disclosure.

FIG. 6A to FIG. 6C are partial embodiments showing a method of motion detection and correction of a medical image according to the present disclosure, with COM in FIG. 6C denoting a center of mass.

FIG. 7 is a partial embodiment showing a method of motion detection and correction of a medical image according to the present disclosure, with COM1 and COM2 denoting center of mass 1 and center of mass 2, respectively.

FIG. 8 is a partial embodiment showing a method of motion detection and correction of a medical image according to the present disclosure.

FIG. 9 is a partial embodiment showing a method of motion detection and correction of a medical image according to the present disclosure.

FIG. 10 is a partial embodiment showing a method of motion detection and correction of a medical image according to the present disclosure.

FIG. 11 is a partial embodiment showing a method of motion detection and correction of a medical image according to the present disclosure.

FIG. 12 is a schematic diagram showing a method of motion detection and correction of a medical image according to an embodiment of the present disclosure.

## DETAILED DESCRIPTION

The following illustrates the implementation of the present disclosure by means of specific embodiments, and other advantages and efficacy of the present disclosure can be easily understood by those skilled in the art from the contents described herein. The structure, proportion, size, etc. of the drawings attached to the present disclosure are only used to match the contents described in the specification for the understanding and reading of those skilled in the art, and are not used to limit the implementation of the present disclosure. Therefore, any modification, alteration, or adjustment, without affecting the efficacy of the present disclosure and the purpose that can be achieved, should still fall within the scope of the technical content described in the present disclosure.

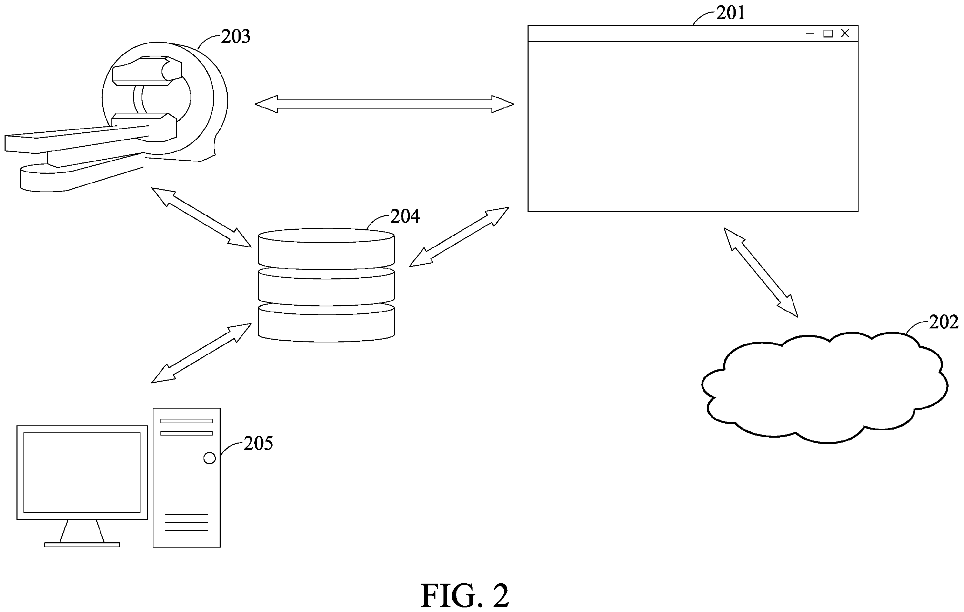

A schematic diagram of a system architecture of the present disclosure for performing motion detection and correction of medical images can be observed from FIG. 2.

In at least one embodiment, a management platform 201 of the present disclosure is used to integrate the processing of medical images, including: receiving and transmitting medical images, providing access to the medical images by a user, and performing optimized processing of the medical images according to the needs of the user. In some embodiments, the management platform 201 may be presented through any suitable user interface such as a web page, an application page, a human-computer interface, etc., and is not specifically limited herein.

In at least one embodiment, an optimization device 202 of the present disclosure is used to perform a background service corresponding to medical image optimization processing (including, motion detection and correction) based on instructions submitted by a user at the management platform 201. In some embodiments, the optimization device 202 of the present disclosure may be any suitable physical computer system, a cloud-based system, etc., and the optimization device 202 may also be implemented with the management platform 201 as an integrated computer system, and is not specifically limited in the present disclosure.

In at least one embodiment, a scanning device 203 of the present disclosure may be any detection device that can take medical images, for example, including but not limited to: a single photon emission computed tomography device, a positron emission tomography device, a magnetic resonance imaging device, a computed tomography device, and the like for performing medical images of the parts of the patient that the patient desires to be detected (for example, including but not limited to: the heart, lungs, coronary arteries, liver, stomach, etc.) for obtaining medical images. In some embodiments, the medical image obtained by the scanning device 203 of the present disclosure includes its corresponding list mode data, and thus facilitates non-instantaneous (e.g., after-access) regression analysis and correction of the captured medical images.

In at least one embodiment, a medical image storage and transmission system 204 of the present disclosure may be any current hospital storage system for storing the medical images acquired at the scanning device 203 described above and/or optimized medical images optimized by the optimization device 202.

In at least one embodiment, an in-office reporting computer 205 of the present disclosure may be any terminal device used by a physician in an in-office setting to provide the physician with access to, or display of, the medical images and/or optimized medical images stored by the medical image storage and transmission system 204.

In at least one embodiment of the present disclosure, the management platform 201, the optimization device 202, the scanning device 203, the medical image storage and transmission system 204 and the in-office reporting computer 205 shown in FIG. 2 are configured to communicate with each other in standard digital imaging and communications in medicine (DICOM), thus providing a high degree of expandability of the present disclosure. In addition, the present disclosure is not limited to the elements described above; for example, depending on operational requirements, any number of the elements described above may be integrated into a single device, or a single management platform 201 and/or optimization device 202 may be designed to support optimization of medical images from multiple scanning devices 203, etc., and the present disclosure is not specifically limited thereto.

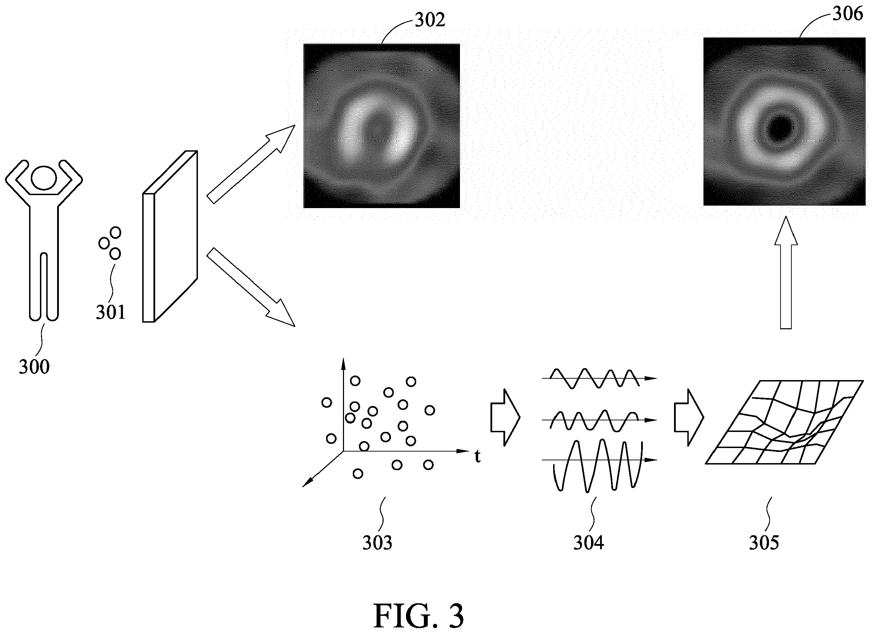

FIG. 3 is a schematic diagram showing a specific embodiment of the present disclosure for performing motion detection and correction of a medical image.

Specifically, in at least one embodiment, the present disclosure may perform motion detection and correction of a medical image of a heart captured using a single photon emission computed tomography (SPECT) scanning device. For example, the scanning device 203 of the present disclosure (e.g., a single-photon emission computed tomography scanning device) includes a CdZnTe (CZT) gamma camera fitted with 19 pinhole collimators and 19 CZT sensors (e.g., CZT elements including $32 \times 32$ pixels) for scanning a heart from the right-slanted anterior view to the left-slanted posterior view and obtaining a correlative SPECT image (i.e., a medical image). In addition, the imaging process of the SPECT image by the scanning device 203 may include the steps of: setting a dual energy window for scanning the heart with asymmetric (e.g., -14% to 23%) and symmetric (e.g., -9% to 9%) settings, respectively; storing list mode data and/or frame mode data corresponding to the SPECT image based on the scanning results; transmitting the list mode data and/or frame mode data to a workstation built into the scanning device 203 in a standard form of DICOM; and resampling the list mode data and/or frame mode data along the short axis, the vertical long axis and the horizontal long axis of the heart for display. However, the description of the specifications, equipment, or means of obtaining medical images to which the scanning device 203 is applicable in the present disclosure is provided only by way of example and is not intended to limit the present disclosure.

In the process schematic shown in FIG. 3, the medical image (shown by the element symbol 302) obtained after $\gamma$ photons 301 released from a patient 300 (who is undergoing myocardial perfusion imaging) is imaged by the scanning device 203 during filming (as in the aforementioned imaging process). At this time, the medical image 302 may be stored directly to the medical image storage and transmission system 204 for access by the in-office reporting computer 205. However, in the event that the medical image 302 may be blurred due to movement of the patient 300 during the scanning, the medical image 302 received from the scanning device 203 may be designated by the management platform 201 to undergo instantaneous motion detection and correction by means of the optimization device 202, including processing procedures such as time dimension segmentation 303, motion center-of-mass analysis 304 and deformation model correction 305, thereby obtaining an optimized medical image 306. Further, when the obtained medical image 302 includes list mode data, the medical image 302 may also be accessed by the management platform 201 to the medical image storage and transmission system 204 when the medical image 302 is found to be ambiguous afterwards, and the optimization device 202 may carry out regression analysis and correction.

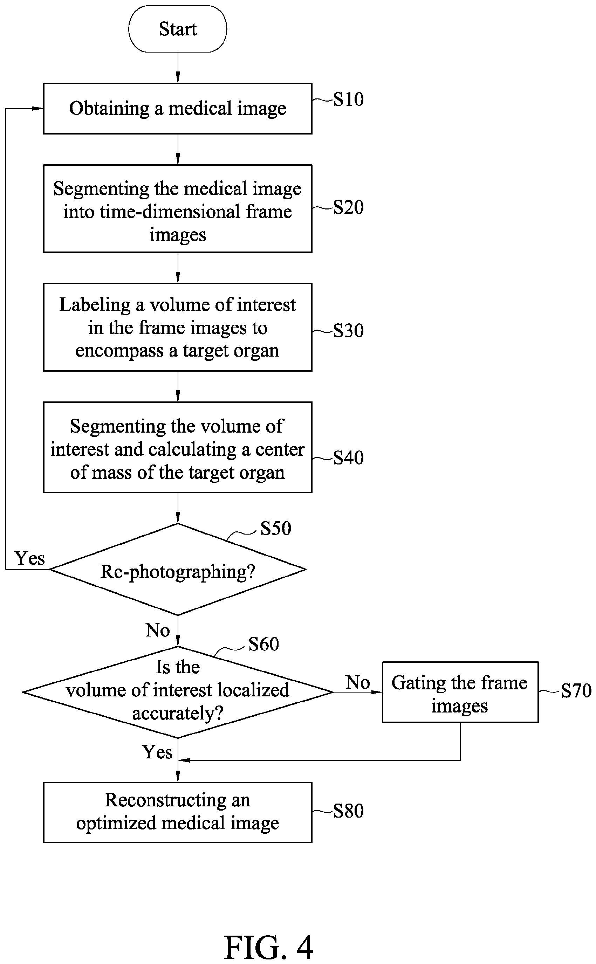

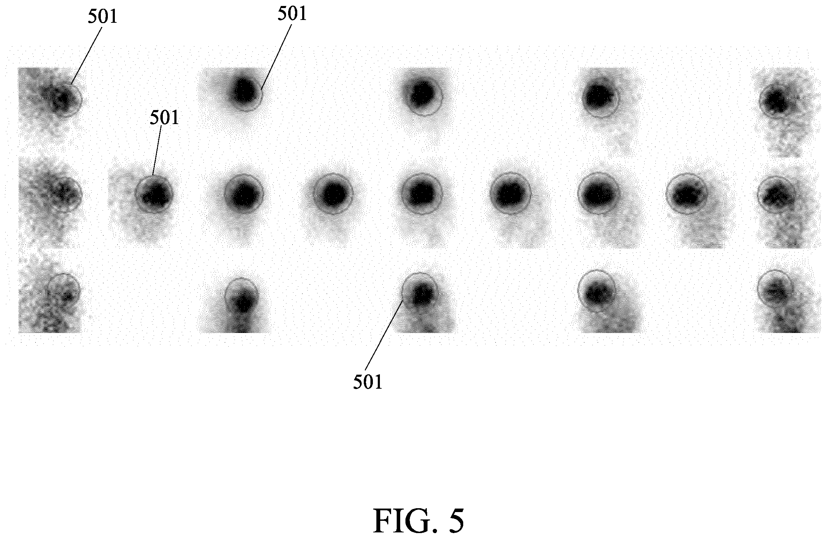

FIG. 4 is a flowchart of the steps for performing motion detection and correction of a medical image (e.g., the optimization device 202 performs the processing procedures of the reference numerals 303 to 305 in FIG. 3) according to the present disclosure, and the embodiments of each of the steps therein may be progressively understood through FIG. 5 to FIG. 12 and the following description.

In at least one embodiment of the present disclosure, at step S10, a medical image for which motion detection and correction is desired (e.g., a set of stress images and rest images of the heart of a particular patient obtained under a single scan) may be selected by a user interface provided by the management platform 201. At this point, said medical images may be obtained instantly by the management platform 201 while the patient is performing a scan at the scanning device 203, or accessed by the management platform 201 to the medical image storage and transmission system 204 when needed.

In at least one embodiment of the present disclosure, at step S20, taking into account the displacement of a target organ (e.g., a heart) captured by the medical image during different phases of respiration and/or heartbeat cycles (or movement of the patient himself) during the capturing period (i.e., events), the optimization device 202 performs the time dimension segmentation 303 shown in FIG. 3, cuts the events contained in the list mode data corresponding to the medical image (e.g., events captured using the aforementioned dual-energy window) into frames having a fixed time dimension (e.g., in units of 500 milliseconds), and imaging said frames by back-projection to an object plane (e.g., the surfaces of the 19 CZT sensors of the aforementioned scanning device 203, which are parallel to the corresponding image planes and intersect the common focal points of the multiple pinhole system), as shown in the imaging results (also referred to subsequently as frame images) for each frame in FIG. 5. In some embodiments, the frames may be cut in a fixed time dimension in units of any value from 100 ms to 500 ms; however, the frames may also be cut in other suitable fixed time dimensions depending on the computing power or operational requirements of the optimization device 202, and the present disclosure is not particularly limited thereto.

In at least one embodiment of the present disclosure, at step S30, as shown in FIG. 5, the optimization device 202 labels a volume of interest 501 containing the target organ (e.g., the heart) in each frame image, thereby learning displacement information such as the position, angle of rotation, and/or axes (e.g., the long and short axes of the heart) of the target organ in each frame image. In some embodiments, the volume of interest 501 including the target organ is spherical; however, the volume of interest 501 may also be labeled in other suitable shapes depending on the shape of the target organ or operational requirements, and the present disclosure is not particularly limited thereto.

In some embodiments of the present disclosure, as an example, the volume of interest is labeled in the myocardial volume of a heart in a frame image, and the labeling of the myocardial volume may be carried out by a physician by a specific application program (e.g., automated software built within the optimization device 202), and practical steps thereof may include: labeling a 3D elliptical sphere passing through the center of the myocardium in a frame image (e.g., the one obtained at the aforementioned step S20); obtaining, from said 3D elliptical sphere, values of 12 spherical parameters including 3D coordinates of the center of the sphere, a radius in three axes (i.e., the axial directions such as the short axis, the vertical long axis, and the horizontal long axis of the heart), an angle of rotation in the three axes, a length ratio and an angle of the cardiac basal plane, and the like; and according to the above spherical parameters, fitting a smooth inner and outer surface inwardly and outwardly, respectively, from the 3D elliptical sphere labeled by the physician by said specific application program through the 3D active contour model, and the frame image of the completed fit of the 3D elliptical sphere is the frame image of the volume of interest including the defined volume of the myocardium.

In some embodiments of the present disclosure, the annotation of the volume of interest 501 by step S30 may also be implemented by a deep learning module (e.g., a deep learning module constructed within the optimization device 202). In an embodiment, the deep learning module includes a convolutional layer, an inverse convolutional layer, a leaky linear rectifier activation layer, residual connections, beating connections, and other major structures, and thousands of sets of framed images of myocardial volumes labeled by a physician (e.g., as previously described for those who are labeled through a specific application program) are used as training data for the deep learning module. A deep learning module that completes training and is capable of accurately predicting the myocardial volume from the unlabeled frame images when inputting may be used for labeling of the volume of interest 501 at step S30.

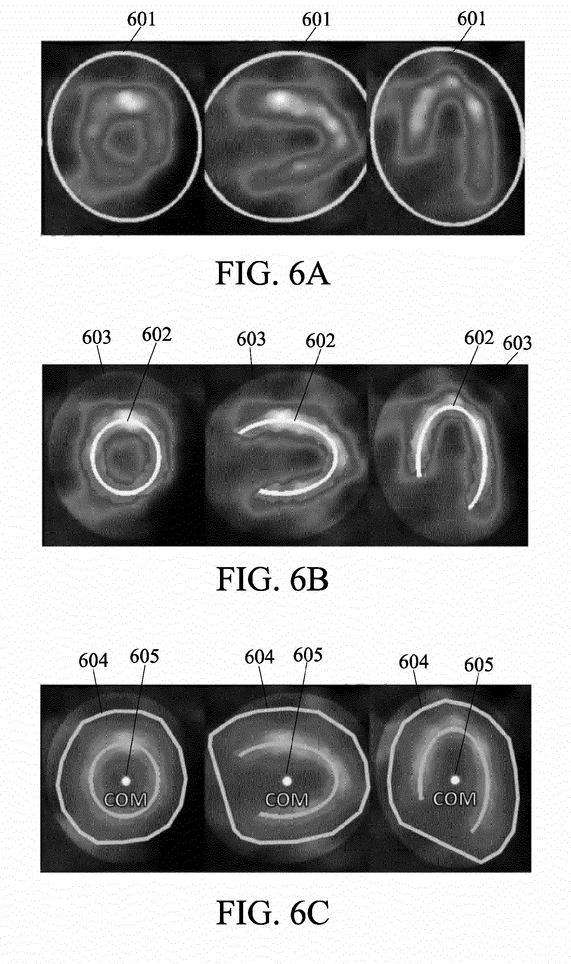

FIG. 6A to FIG. 6C are schematic diagrams of stages of applying the deep learning module to labeling the volume of interest 501. In at least one embodiment of the present disclosure, the trained deep learning module may first receive a frame image generated at step S20 (such as a cut-away picture of the heart in the dimensions of its short axis, vertical long-axis, and horizontal long-axis as shown in FIG. 6A). In some embodiments of the present disclosure, the deep learning module recognizes a volume 601 where the heart is located in the frame image (as shown in FIG. 6A circling a range of a positive ellipse [with a rotation angle of zero] at least 5 cm away from the segmented volume of the heart with the solid line volume 601), thereby recognizing a binary segmented volume 603 containing a volume of the myocardium (as the volume labeled by 603 in FIG. 6B). Subsequently, the deep learning module blurs this binary segmented volume 603 to generate a soft mask, and further applies this soft mask to the original frame image, thereby excluding image activity in volumes other than the myocardial volume in the frame image. Finally, based on the soft mask, the deep learning module fits a myocardial volume 602 on the frame image with an elliptical sphere (as shown by the elliptical sphere cross section corresponding to the solid line indicated by 602 in FIG. 6B) and expands the elliptical sphere radius outwardly by 2 centimeters to generate a new elliptical sphere representing a volume of interest 604 (as shown by the volume corresponding to the solid line indicated by 604 in FIG. 6C).

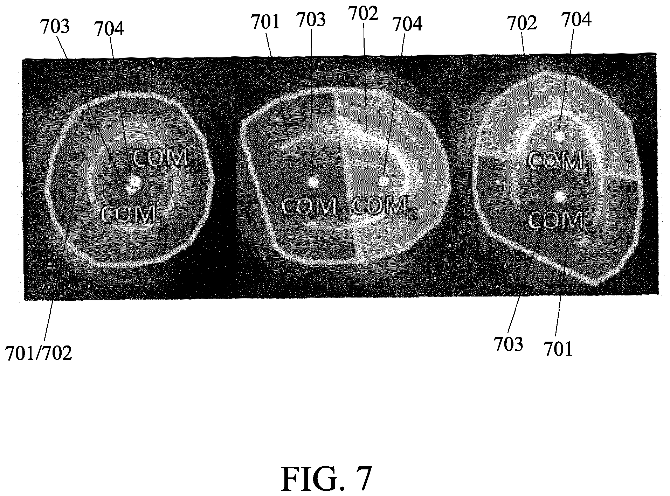

In at least one embodiment of the present disclosure, at step S40, the optimization device 202 performs the motion center-of-mass analysis 304 shown in FIG. 3 with respect to the target organ within the volume of interest labeled at step S30. In some embodiments of the present disclosure, firstly, in order to accurately observe the displacement and rotation of the target organ (e.g., the heart) in each frame image, the volume of interest in each frame image will be divided into two copies. An implementation of segmenting a volume of interest may be observed with reference to the difference between FIG. 6C and FIG. 7. For example, the volume of interest 604 of each frame image labeled in FIG. 6C is segmented into two along the long axis of the heart in this step S40 to form sub-volumes of interest 701, 702 about the heart as shown in FIG. 7, wherein the observed sub-volumes of interest 701, 702 are presented in an overlapping manner because the left side of FIG. 7 is a schematic diagram of the heart in the short-axis dimension thereof. Subsequently, by means of the resulting two sub-volumes of interest 701, 702 of the target organ, their respective centers of mass 703, 704 can be calculated, thereby assisting in tracking the movement of the target organ in each frame image, and thus obtaining a motion curve of the target organ during the filming.

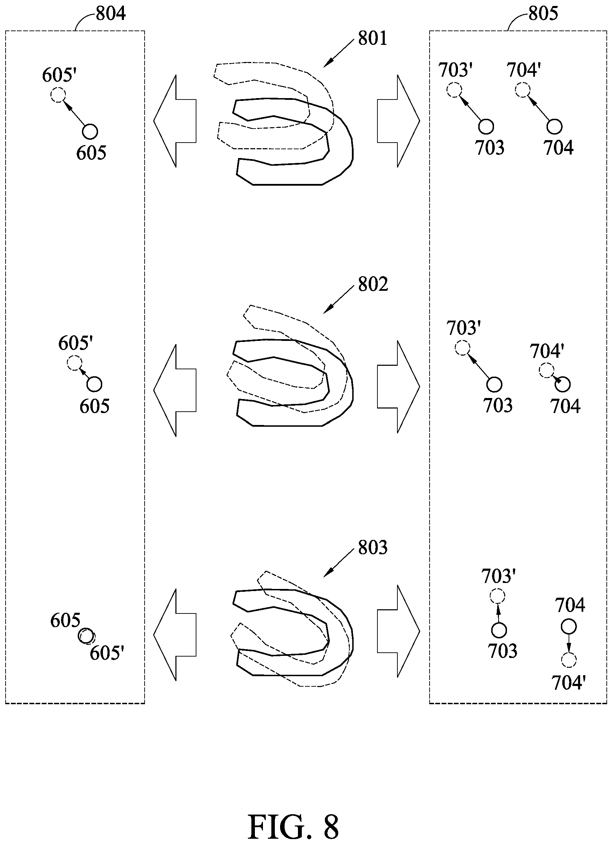

Compared to the volume of interest 604 shown in FIG. 6C with only one center of mass 605, the advantage of segmenting the volume of interest 604 into two sub-volumes of interest 701, 702 and then performing motion center-of-mass analysis by the obtained two centers of mass 703, 704 shown in FIG. 7 is illustrated in FIG. 8, wherein 801 to 803 represent the cases when the target organ (e.g., the heart) translates (801), translates and rotates (802), rotates (803), etc. (i.e., changes from a solid line pattern to a dashed line pattern), respectively, and 804 and 805 label the effects of observing the above cases 801 to 803 with a single center of mass 605 and two centers of mass 703, 704, respectively. As can be seen, although in the case where only translation 801 of the target organ occurs it can be clearly traced regardless of whether a single center of mass 605 or two centers of mass 703, 704 are used (e.g., the change in movement of center of mass 605 to 605' and the change in movement of centers of mass 703, 704 to 703', 704'), in the case where the target organ is translating and rotating 802 or rotating 803, however, the tracking effect observed with only a single center of mass 605 is significantly less effective than the tracking effect observed with two centers of mass 703, 704, especially when the target organ is only rotating 803, the change of center of mass 605 before and after the rotation (overlapping with 605') is not obvious, resulting in the inability to track the target organ.

However, the present disclosure is not limited to the manner of analyzing a moving center of mass as described above. For example, one of ordinary skill in the art should be able to understand that the manner of dividing the volume of interest 604 into sub-volumes of interest 701, 702 may also be carried out along the short-axis of the heart (in the direction of the long-axis). Alternatively, according to the needs (e.g., according to the shape characteristics of the target organ), the volume of interest 604 is divided into more than two sub-volumes of interest, and the displacement and rotation of the target organ are observed in a manner with more than two centers of mass.

Immediately after step S40, when the volume of interest 604 is divided into sub-volumes of interest 701, 702, the three-dimensional coordinates corresponding to the centers of mass 703, 704 in each frame image (e.g., denoted by "$(x1,y1,z1), (x2,y2,z2)$") are computed as a descriptive value of each frame image. In some embodiments of the present disclosure, the descriptive value of each frame image may be downscaled (down dimensionalized) using principal component analysis (PCA), and the maximum feature is obtained from the downscaled descriptive value(s) to be used as the movement/rotation signals of the target organ in each frame image. In some embodiments of the present disclosure, each frame image may also be filtered based on the movement/rotation signals of the target organ to filter out the overly high-frequency spurious signals, thereby calculating the motion curve of the target organ during the filming.

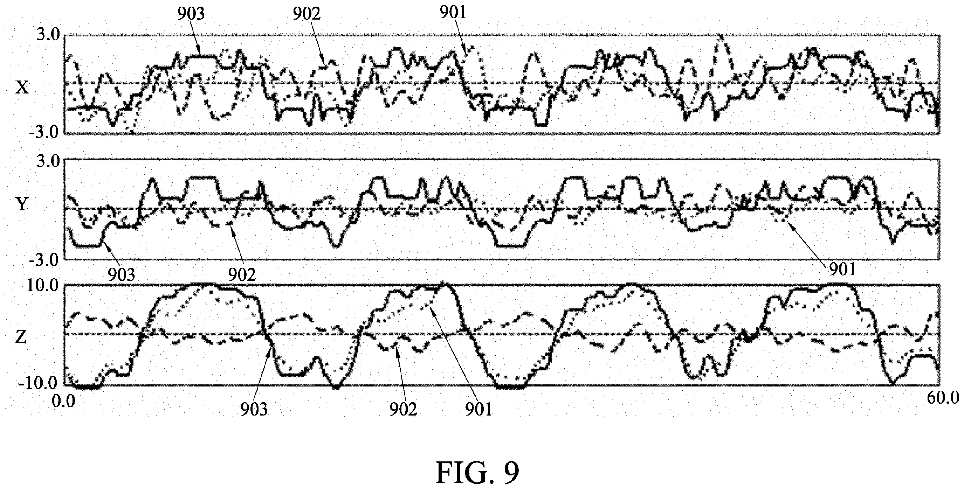

In at least one embodiment of the present disclosure, the motion curve of the target organ during filming is shown in FIG. 9, which depicts the motion curve of the detected target organ (heart) relative to the left and right sides of the human body (X-axis, schematic diagram of the upper layer of FIG. 9), the ventral and dorsal sides of the human body (Y-axis, schematic diagram of the middle layer of FIG. 9), and the head and tail of the human body (Z-axis, schematic diagram of the lower layer of FIG. 9) during the filming, wherein a curve 901 shown in FIG. 9 (indicated by the dotted dashed line) represents the relative position change of the center of mass 703 or 704 in each frame image in the above X-axis, γ-axis and Z-axis, a curve 902 shown in FIG. 9 (indicated by the short dashed line) represents the rotation signal of the center of mass 703 or 704 in the above X-axis, γ-axis and Z-axis calculated according to the curve 901, and a curve 903 shown in FIG. 9 (indicated by the solid line) represents the more precise position change of the center of mass 703 or 704 obtained by grouping and accumulating the images of each frame image.

In at least one embodiment of the present disclosure, at step S50, in order to ensure the benefit of the subsequent step of correcting the medical image, the optimization device 202 may first determine whether the degree of movement of the target organ is too large based on the aforementioned motion curve of the target organ, and in the case where the degree of movement of the target organ is too large (e.g., when the amplitude of the motion curve of the target organ is greater than 50 mm), the radiologist may request the patient to undergo a new scanning and repeat the steps S10 to S40 in order to obtain a new motion curve of the target organ. If the re-scanning is not required, the subsequent steps may be continued to perform the deformation model correction of the medical image.

The above steps S10 to S50 are used to complete the procedure of motion detection of the medical image, and based on the results of the motion detection, the optimization device 202 may proceed to perform the deformation model correction 305 shown in FIG. 3 on the medical image, as described in steps S60 to S80 below.

Given that the aforementioned motion curve is the average data of the positions of the volumes of interest (based on the center of mass) in all frame images, the optimization device 202 first confirms at step S60 whether the position of the volume of interest in each frame image accurately corresponds to the said motion curve, and if the volume of interest in each frame image does not accurately correspond to the motion curve, the gating of step S70 may be performed. Conversely, each frame image may be directly subjected to the optimized reconstruction of the medical image at step S80. In some embodiments of the present disclosure, regardless of whether the volume of interest of each frame image corresponds accurately to the motion curve or not, gating each frame image directly after step S70 can help to further grasp the movement of the target organ and reduce the time taken to optimize the reconstruction of the medical image in step S80.

In at least one embodiment of the present disclosure, the gating described in step S70 is used to form gated set images by integrating the various frame images that have similar temporal and/or positional relationships based on the motion curve of the target organ (e.g., the heart) calculated in step S40.

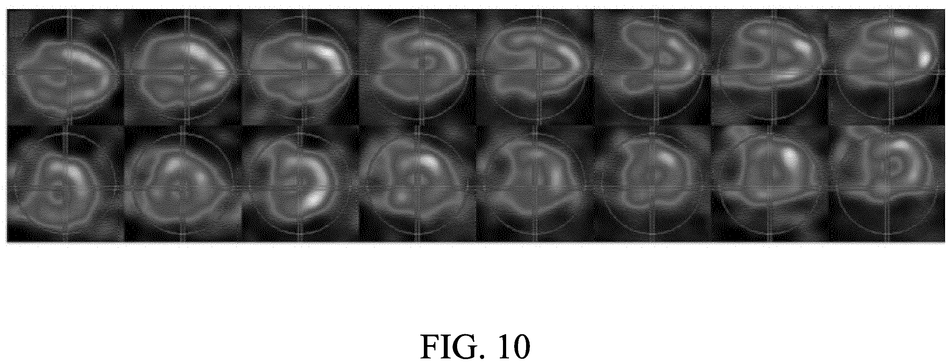

FIG. 10 is an embodiment of the present disclosure in which each frame image of a medical image of the heart is organized into eight gated sets, wherein the eight gated sets in the upper row are gated set images viewed with the vertical long axis of the heart, while the eight gated sets in the lower row are gated set images viewed with the short axis of the heart. In addition, considering that the said motion curve may correspond to the regular motion of the human body's respiration and/or heartbeat, in the case where there is no strenuous motion of the patient occurs during the scanning period, the eight gated set images can also correspond to the various cyclic phases of respiration and/or heartbeat, as shown in FIG. 10 from the left to the right for the inhalation/exhalation respiration cyclic phases, and each of gated set images has an iterative displacement relationship relative to the gated set image of the end-of-inhalation phase (the leftmost gated set image) when view on the head-tail axis, the left-right axis and/or ventral-dorsal of the human body, and thus step S70 is also referred to as respiratory gating or heartbeat gating.

In some embodiments of the present disclosure, FIG. 10 schematically depicts an embodiment in which all frame images from a medical image of the heart are grouped into eight gated sets based on the phases of the human respiratory cycle (i.e., each gated set includes 12.5% of the total number of frame images); however, the number of gated sets may also be increased or decreased, or an additional set of gated set images may be formed by additionally considering the human cardiac cycle, or a set of gated set images may be formed by integrally considering the phases of the respiratory and cardiac cycles, according to the need for the optimization effect of the correction of the medical image or the need for the operation, which is not specifically limited herein.

In at least one embodiment of the present disclosure, at step S80, the reconstruction optimization of the medical image is performed based on the frames images that have been accurately positioned (i.e., each frame image of the medical image that has not been processed at step S70) or the gated set images that are gated at step S70. The execution of the reconstruction optimization of the medical image is mainly based on a reference object (e.g., a frame image or gated set images judged to be in the end-inspiratory phase, the isovolumetric systolic phase of a cardiac cycle, or a frame image or gated set images that meet(s) both of the foregoing) in each frame image or gated set images as the standard for motion compensation of the remaining frame images or the gated set images, and the aforesaid motion curves with respect to the head-tail axis, the left-right axis and/or the ventral-dorsal axis of the human body are used to as a reference for adjusting the frame images or the gated set images when performing the above-mentioned motion compensation (including the adjustment operation of rotating, displacement, scaling, deforming, and so on), and the correlation coefficients between the reconstructed optimized medical image and the reference object are used for observing the degree of completion of reconstruction optimization of the medical image.

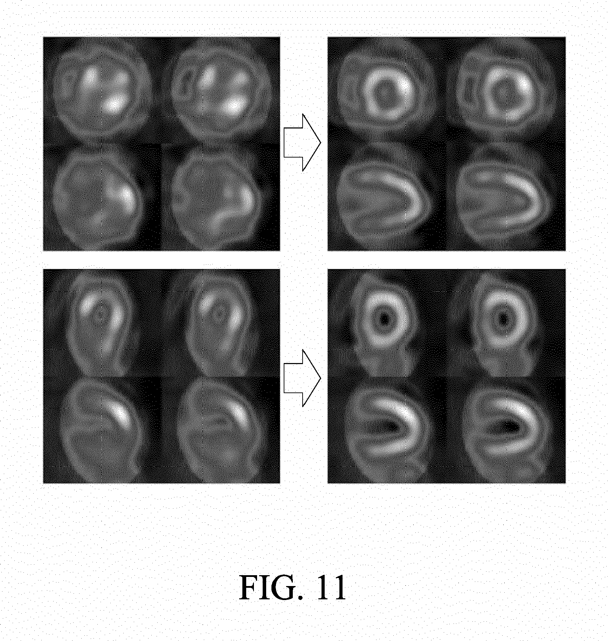

For example, when optimizing the reconstruction of the medical image of the heart in consideration of the respiratory cycle phase of the human body, the optimization device 202 selects a frame image or gated set images at the end of the inspiratory phase as a reference object among the corresponding frame image or the gated set images of said medical image and adjusts all the pixels contained in the rest of the frame images or the gated set images under a reference motion curve relative to said reference object, according to their three-dimensional coordinates in the head-tail axis, left-right axis and/or ventral-dorsal axis of the human body (i.e., the aforementioned adjustment operations of rotating, displacement, scaling, etc.), thereby integrating each of the adjusted frame images or gated set images in order to reconstruct them as an optimized medical image and iteratively executing the reconstruction and optimization of said medical image until the optimized medical image reaches the most similarity with the reference object (e.g., the correlation coefficient reaches the maximum value or the root mean square error reaches the minimum value), which represents that the motion compensation of this medical image has been completed, and then the motion compensation effect can be achieved as shown in FIG. 11 from the left side of the image (without reconstruction optimization) to the right side of the image (with reconstruction optimization completed).

In some embodiments of the present disclosure, the motion compensation performed at step S80 is performed by incorporating the motion compensation procedure into a maximum a posteriori expectation maximization (MAPEM) algorithm, which may be expressed in the form of the following mathematical equation:

[mathematical equation 1]

$$x_j^{next} = x_j^{current} \frac{1}{\sum_k \sum_i a_{ij}^k + \beta \frac{\partial U}{\partial x_j}} \sum_k \sum_i a_{ij}^k \frac{p_i^k}{\sum_j a_{ij}^k x_j^{current}}$$

wherein $p_\square^k$ represents the projection result of the kth frame image or gated set images of this medical image, i represents the index value of each pixel in said projection result, $a_\square^k$ : represents the system matrix for modeling the kth frame image or gated set images of this medical image (i.e., the new system matrix adjusted by compensating the original system matrix based on the movement, rotation, translation, or deformation information of the kth frame image or gated set images), j represents an adjusted index value of each pixel in said projection result, $\frac{\partial U}{\partial x_j}$ represents a partial derivative of the median square root a priori energy function, $\beta$ represents an a priori adjustable factor, $x^{current}_{\square}$ represents a reconstruction result of the medical image estimated for the current iteration loop (i.e., the optimized medical image for the current iteration loop), and $x^{next}_{\square}$ represents a reconstruction result of the medical image estimated for the next iteration loop (i.e., the optimized medical image for the next iteration loop). In some embodiments of the present disclosure, the number of iterations for the optimized reconstruction of the medical image is preset to be 70 (i.e., the maximum value of *next* is set to be 70); however, more or less iterations may be set depending on the operational requirements, and there is no particular limitation in the present disclosure.

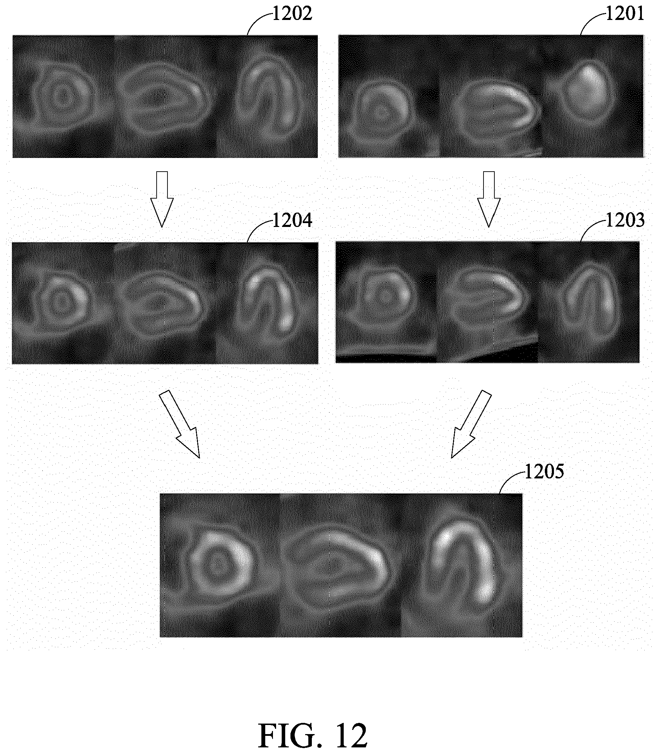

FIG. 12 is a schematic diagram showing the effect of the optimization device 202 after performing steps S10 to S80 described above on the medical image. For example, in at least one embodiment of the present disclosure, the optimization device 202 analyzes medical images of a target organ (e.g., the heart) by parsing the medical images into frame image (e.g., frame images 1201 corresponding to a certain respiratory cycle phase shown in FIG. 12 and/or frame images 1202 corresponding to a certain heartbeat cycle phase shown in FIG. 12), and subsequently considers a motion curve of the target organ so as to adjust each of the frame images (or the gated set images after gating) relative to the head-tail axis, the left-right axis, and/or the ventral-dorsal axis of the human body (e.g., frame images 1203 corresponding to a certain respiratory cycle phase shown in FIG. 12 and/or frame images 1204 corresponding to a certain heartbeat cycle phase shown in FIG. 12), and an optimized medical image 1205 is then reconstructed. Herein, the optimization device 202 may directly display the reconstructed optimized medical image 1205 on a user interface of the management platform 201, or store the reconstructed optimized medical image 1205 through the management platform 201 to the medical image storage and transmission system 204, and thereby make it available for access by a physician via the in-office reporting computer 205.

The present disclosure further provides a computer-readable medium, applied in a computer or computing device having a processor and/or a memory, which stores instructions such that the computer or computing device may perform, via the processor (e.g., central processing unit [CPU], graphics processing unit [GPU], etc.) and/or the memory, a method of motion detection and correction of a medical image as described above via the instructions.

In summary, the method, system, and computer-readable medium for motion detection and correction of medical images in the present disclosure can be used to segment a medical image of a target organ into a plurality of frame images in accordance with list mode data, analyze a plurality of centers of mass of a volume of interest in said plurality of frame images in order to compute a motion curve of the target organ during scanning, and then optimize the reconstruction of a medical image on the basis of said motion curve. As such, it is possible to take into account the movement of a human organ or a lesion without installing an additional monitoring device in order to perform motion detection and correction of the medical image.

The above embodiments are merely illustrative of the effects of the present disclosure and are not intended to limit the scope of the present disclosure, and any person skilled in the art may modify or change the above embodiments without violating the scope of the present disclosure. Therefore, the scope of protection of the present disclosure shall be as set forth in the claims.

**Claims**

1. A system of motion detection and correction of a medical image, comprising:

   a management platform providing a user interface to submit instructions for performing optimization processing on a medical image of a target organ, wherein said medical image corresponds to list mode data; and
   an optimization device performing said optimization processing of said medical image in accordance with said instructions, wherein said optimization processing comprises:

      segmenting said list mode data corresponding to said medical image into a plurality of frames having a fixed time dimension, and imaging each said frame as a multi-frame image;
      labeling a volume of interest in each said frame image to encompass said target organ;
      calculating a motion curve of said target organ based on said volume of interest of each said frame image;
      reconstructing said medical image based on said motion curve; and
      displaying said reconstructed medical image on said user interface.

2. The system of claim 1, wherein said optimization device comprises a deep learning module, and labeling the volume of interest in each of said frame images comprises:

> recognizing a binary segmented volume containing said target organ in each said frame image by said deep learning module;
> blurring each said binary segmented volume by said deep learning module to generate a soft mask;
> applying each said soft mask to each said frame image by said deep learning module;
> fitting said target organ in each said frame image with an initial elliptical sphere based on each said soft mask by said deep learning module; and
> expanding each said initial elliptical sphere by a predetermined distance outwardly from a radius thereof by said deep learning module to generate an elliptical sphere representing said volume of interest.

3. The system of claim 1, wherein calculating the motion curve of said target organ based on said volume of interest of each said frame image comprises:

> dividing said volume of interest into a first sub-volume of interest and a second sub-volume of interest;
> extracting three-dimensional coordinates of a first center of mass of said first sub-volume of interest and a second center of mass of said second sub-volume of interest, respectively, to be used as descriptive values of said frame images;
> down dimensionalizing said descriptive values of each said frame image by principal component analysis, and using a largest feature of said descriptive values after down dimensionalizing as a movement and/or rotation signal of said target organ; and
> grouping and filtering each said frame image based on each said movement and/or rotation signal to calculate said motion curve.

4. The system of claim 3, wherein said target organ is a heart, and said dividing said volume of interest into a first sub-volume of interest and a second sub-volume of interest is along a long axis of said heart in a short-axis direction.

5. The system of claim 1, wherein said motion curve is plotted relative to any one of a head-tail axis, a left-right axis, and a ventral-dorsal axis of a human body, and reconstructing said medical image based on said motion curve comprises:

> selecting a reference object from each said frame image;
> performing motion compensation of each said frame image using said motion curve as a reference, wherein said motion compensation comprises:
>
> > performing an adjustment operation of all pixels contained in each said frame image other than said reference object in any one of said head-tail axis, said left-right axis, and said ventral-dorsal axis according to three-dimensional coordinates thereof with respect to said reference object; and
> > repeating said adjustment operation of each said frame image until correlation coefficient between integration of each said frame image after said adjustment operation and said reference object reaching a maximum value; and
>
> integrating each said frame image after said motion compensation to reconstruct said medical image.

6. The system of claim 3, further comprising:
after calculating the motion curve of said target organ based on each said center of mass, and each said volume of interest of each said frame image does not accurately correspond to said motion curve, said optimization device performing said optimization processing alternatively comprising performing gating of each said frame image, wherein said gating is used to assemble a predetermined number of gated set images with similar time and/or positional relationships among said frame images, and said time and/or positional relationships are defined by cyclic phases of human respiration and/or heartbeat.

7. The system of claim 6, wherein said motion curve is plotted relative to any one of a head-tail axis, a left-right axis, and a ventral-dorsal axis of a human body, and reconstructing said medical image based on said motion curve comprises:

> selecting a reference object from each said gated set image;

performing motion compensation of each said gated set image using said motion curve as a reference, wherein said motion compensation of each said gated set image comprises:

performing an adjustment operation of all pixels contained in each said gated set image other than said reference object in any one of said head-tail axis, said left-right axis, and said ventral-dorsal axis according to three-dimensional coordinates thereof with respect to said reference object; and
repeating said adjustment operation of each said gated set image until correlation coefficient between integration of each said gated set image after said adjustment operation and said reference object reaching a maximum value; and

integrating each said gated set image after said motion compensation to reconstruct said medical image.

8. The system of claim 1, wherein said fixed time dimension is measured in units of 100 milliseconds to 500 milliseconds.

9. The system of claim 1, further comprising:

a scanning device photographing said target organ to obtain said medical image, wherein said scanning device is selected from a group consisting of a single photon emission computed tomography device, a positron emission tomography device, a magnetic resonance imaging device, and a computed tomography device or a combination thereof;
a medical image storage and transmission system storing said medical image and said medical image as reconstructed; and
an in-office reporting computer accessing or displaying said medical image and said medical image as reconstructed.

10. A method of motion detection and correction of a medical image, comprising:

obtaining a medical image of a target organ, wherein said medical image corresponds to list mode data;
segmenting said list mode data corresponding to said medical image into a plurality of frames having a fixed time dimension, and imaging each said frame as a multi-frame image;
labeling a volume of interest in each said frame image to encompass said target organ;
calculating a motion curve of said target organ based on said volume of interest of each said frame image; and
reconstructing said medical image based on said motion curve.

11. The method of claim 10, wherein labeling said volume of interest in each said frame image to encompass said target organ comprises:

recognizing a binary segmented volume containing said target organ in each said frame image by a deep learning module;
blurring each said binary segmented volume by said deep learning module to generate a soft mask;
applying each said soft mask to each said frame image by said deep learning module;
fitting said target organ in each said frame image with an initial elliptical sphere based on each said soft mask by said deep learning module; and
expanding each said initial elliptical sphere by a predetermined distance outwardly from a radius thereof by said deep learning module to generate an elliptical sphere representing said volume of interest.

12. The method of claim 10, wherein calculating the motion curve of said target organ based on said volume of interest of each said frame image comprises:

dividing said volume of interest into a first sub-volume of interest and a second sub-volume of interest;
extracting three-dimensional coordinates of a first center of mass of said first sub-volume of interest and a second center of mass of said second sub-volume of interest, respectively, to be used as descriptive values of said frame images;
down dimensionalizing said descriptive values of each said frame image by principal component analysis, and using a largest feature of said descriptive values after down dimensionalizing as a movement and/or rotation signal of said target organ; and
grouping and filtering each said frame image based on each said movement and/or rotation signal to calculate said motion curve.

13. The method of claim 12, wherein said target organ is a heart, and said dividing said volume of interest into a first sub-volume of interest and a second sub-volume of interest is along a long axis of said heart in a short-axis direction.

14. The method of claim 10, wherein said motion curve is plotted relative to any one of a head-tail axis, a left-right axis, and a ventral-dorsal axis of a human body, and reconstructing said medical image based on said motion curve comprises:

selecting a reference object from each said frame image;
performing motion compensation of each said frame image using said motion curve as a reference, wherein said motion compensation comprises:

performing an adjustment operation of all pixels contained in each said frame image other than said reference object in any one of said head-tail axis, said left-right axis, and said ventral-dorsal axis according to three-dimensional coordinates thereof with respect to said reference object; and
repeating said adjustment operation of each said frame image until correlation coefficient between integration of each said frame image after said adjustment operation and said reference object reaching a maximum value; and

integrating each said frame image after said motion compensation to reconstruct said medical image.

15. The method of claim 12, wherein after calculating the motion curve of said target organ based on each said center of mass, and each said volume of interest of each said frame image does not accurately correspond to said motion curve, gating of each said frame image is performed, wherein said gating is used to assemble a predetermined number of gated set images with similar time and/or positional relationships among said frame images, and said time and/or positional relationships are defined by cyclic phases of human respiration and/or heartbeat.

16. The method of claim 15, wherein said motion curve is plotted relative to any one of a head-tail axis, a left-right axis, and a ventral-dorsal axis of a human body, and reconstructing said medical image based on said motion curve comprises:

selecting a reference object from each said gated set image;
performing motion compensation of each said gated set image using said motion curve as a reference, wherein said motion compensation of each said gated set image comprises:

performing an adjustment operation of all pixels contained in each said gated set image other than said reference object in any one of said head-tail axis, said left-right axis, and said ventral-dorsal axis according to three-dimensional coordinates thereof with respect to said reference object; and
repeating said adjustment operation of each said gated set image until correlation coefficient between integration of each said gated set image after said adjustment operation and said reference object reaching a maximum value; and

integrating each said gated set image after said motion compensation to reconstruct said medical image.

17. The method of claim 10, wherein said medical image is obtained by photographing said target organ by a scanning device, and said scanning device is selected from a group consisting of a single photon emission computed tomography device, a positron emission tomography device, a magnetic resonance imaging device, and a computed tomography device or a combination thereof.

18. The method of claim 10, wherein said fixed time dimension is measured in units of 100 milliseconds to 500 milliseconds.

19. A computer-readable storage medium applied in a computer and having instructions to perform the method of motion detection and correction of said medical image as claimed in any one of claims 10 to 18.

FIG. 1

FIG. 2

EP 4 446 973 A1

FIG. 3

Start

Obtaining a medical image ⌐S10

Segmenting the medical image into time-dimensional frame images ⌐S20

Labeling a volume of interest in the frame images to encompass a target organ ⌐S30

Segmenting the volume of interest and calculating a center of mass of the target organ ⌐S40

⌐S50

Yes

Re-photographing?

No

⌐S60

Is the volume of interest localized accurately?

No → Gating the frame images ⌐S70

Yes

Reconstructing an optimized medical image ⌐S80

FIG. 4

FIG. 5

**FIG. 6A**

**FIG. 6B**

**FIG. 6C**

FIG. 7

FIG. 8

FIG. 9

EP 4 446 973 A1

FIG. 10

FIG. 11

FIG. 12

# EP 4 446 973 A1

## INTERNATIONAL SEARCH REPORT

| | International application No. |
|---|---|
| | **PCT/CN2022/136636** |

### A. CLASSIFICATION OF SUBJECT MATTER

G06T5/00(2006.01)i

According to International Patent Classification (IPC) or to both national classification and IPC

### B. FIELDS SEARCHED

Minimum documentation searched (classification system followed by classification symbols)

IPC: G06T

Documentation searched other than minimum documentation to the extent that such documents are included in the fields searched

Electronic data base consulted during the international search (name of data base and, where practicable, search terms used)

VEN, CNTXT, WOTXT, EPTXT, USTXT, BAIDU, CNKI, IEEE: 医学, 影像, 图像, 移动, 平移, 旋转, 运动, 分割, 区域, 校正, 补偿, medical, imagery, image, movement, rotation, motion, segmentation, region, correction, compensation

### C. DOCUMENTS CONSIDERED TO BE RELEVANT

| Category* | Citation of document, with indication, where appropriate, of the relevant passages | Relevant to claim No. |
|---|---|---|
| X | CN 101454801 A (KONINKL PHILIPS ELECTRONICS N.V.) 10 June 2009 (2009-06-10) description, page 1, last paragraph-page 9, paragraph 2 | 1-2, 5, 8-11, 14, 17-19 |
| X | CN 102067176 A (KONINKL PHILIPS ELECTRONICS N.V.) 18 May 2011 (2011-05-18) description, paragraphs [0028]-[0069] | 1-2, 5, 8-11, 14, 17-19 |
| A | CN 101702232 A (KUNMING UNIVERSITY OF SCIENCE AND TECHNOLOGY) 05 May 2010 (2010-05-05) entire document | 1-19 |
| A | CN 107569251 A (SHANGHAI UNITED IMAGING HEALTHCARE CO., LTD.) 12 January 2018 (2018-01-12) entire document | 1-19 |
| A | CN 110619639 A (SUZHOU TONGDIAO MEDICAL TECHNOLOGY CO., LTD. et al.) 27 December 2019 (2019-12-27) entire document | 1-19 |
| A | US 2021104037 A1 (SIEMENS MEDICAL SOLUTIONS USA, INC.) 08 April 2021 (2021-04-08) entire document | 1-19 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **23 February 2023** | **28 February 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
Information on patent family members

| International application No. |
| --- |
| **PCT/CN2022/136636** |

| Patent document cited in search report | | | Publication date (day/month/year) | Patent family member(s) | | | Publication date (day/month/year) |
| --- | --- | --- | --- | --- | --- | --- | --- |
| CN | 101454801 | A | 10 June 2009 | EP | 1991959 | A2 | 19 November 2008 |
| | | | | EP | 1991959 | B1 | 30 August 2017 |
| | | | | JP | 2009528139 | A | 06 August 2009 |
| | | | | JP | 5254810 | B2 | 07 August 2013 |
| | | | | WO | 2007100955 | A2 | 07 September 2007 |
| | | | | WO | 2007100955 | A3 | 07 August 2008 |
| | | | | WO | 2007100955 | A8 | 02 April 2009 |
| | | | | US | 2010166274 | A1 | 01 July 2010 |
| | | | | US | 8144962 | B2 | 27 March 2012 |
| | | | | CN | 101454801 | B | 05 December 2012 |
| CN | 102067176 | A | 18 May 2011 | US | 2011081068 | A1 | 07 April 2011 |
| | | | | US | 8331639 | B2 | 11 December 2012 |
| | | | | EP | 2291827 | A2 | 09 March 2011 |
| | | | | EP | 2291827 | B1 | 14 November 2012 |
| | | | | WO | 2009153683 | A2 | 23 December 2009 |
| | | | | WO | 2009153683 | A3 | 11 February 2010 |
| | | | | CN | 102067176 | B | 30 October 2013 |
| CN | 101702232 | A | 05 May 2010 | None | | | |
| CN | 107569251 | A | 12 January 2018 | None | | | |
| CN | 110619639 | A | 27 December 2019 | None | | | |
| US | 2021104037 | A1 | 08 April 2021 | US | 11270434 | B2 | 08 March 2022 |

Form PCT/ISA/210 (patent family annex) (July 2022)